# DEMANDE DE BREVET EUROPEEN

(11) **EP 1 386 610 A1**
(43) Date de publication de la demande: **04.02.2004**
(21) Numéro de dépôt: 03291881.5
(22) Date de dépôt: 29.07.2003
(51) Int. Cl.: A61K 31/557, A61K 31/558, A61P 3/04, A61P 43/00

(54) **Médicament contenant le 12-HETE ou le 11,12-EET**

(30) Priorité: 02.08.2002 FR 0209911
(71) Demandeur: LABORATOIRES GOEMAR S.A., 35400 Saint-Malo (FR)
(72) Inventeur: Salaun, Jean-Pierre, 29870 Landéda (FR); Plee-Gautier, Emmanuelle, 29200 Brest (FR); Berthou, François, 29200 Brest (FR); Yvin, Jean-Claude, 35400 Saint Malo (FR)
(74) Mandataire: Koch, Gustave

(57) **Abrégé**

Médicament caractérisé par le fait qu'il comporte en tant que substance active une quantité efficace d'au moins l'un des métabolites du groupe comprenant le 12-HETE et le 11,12-EET, dans le but d'inhiber la différenciation des fibroblastes en adipocytes pour lutter contre le développement de certaines formes d'obésité ou de cellulite sous-cutanée.

## Description

L'invention a pour objet un nouveau médicament.

Elle vise également un procédé pour le préparer et son utilisation dans le lutte contre la différenciation des fibroblastes en adipocytes à partir desquels se forme la couche graisseuse dans l'organisme humain ou animal.

Il est connu que la cascade d'oxydation de l'acide arachidonique chez les mammifères aboutit à des métabolites dont notamment
- la 15-déoxy-Δ^{12,14} - prostaglandine J2 ou PGJ2 sous l'action de la cyclooxygénase,
- l'acide 12-hydroxy-éicosatétraénoïque ou 12-HETE sous l'action de la lipoxygenase et
- l'acide 11,12-époxy-eicosatrienoïque ou 11,12-EET sous l'action du cytochrome P450 de la famille CYP2C.

L'acide 12-hydroxy-cicosatétraénoïque existe sous forme de deux stéréoisomères, à savoir 12(S)-HETE et 12(R)-HETE.

L'action de la 12-lipoxygenase fournit la forme majeure à savoir le 12(S)-HETE.

Le stéréoisomère 12(R)-HETE peut être obtenu par réduction du 11,12-EET.

Il est également connu que la PGJ2 est un ligand d'un récepteur nucléaire de la famille des Peroxisome Proliferator Activated Receptors (P.P.A.R.s). La PGJ2 contrôle la différenciation des fibroblastes pré-adipocytes c'est-à-dire la transformation de ces cellules en adipocytes. Cette prostaglandine PGJ2 accélère la différenciation en question à des concentrations de 10⁻⁵ à 10⁻⁶ M.

Or, l'accumulation des adipocytes conduit à la formation de la couche graisseuse entre l'épiderme et les structures sous-jacentes.

On rappelle à cet égard que la différenciation des fibroblastes en adipocytes se traduit par la transformation des premiers qui sont des cellules longilignes dépourvues de globules lipidiques en cellules très arrondies, les adipocytes, qui accumulent de grandes quantités de lipides, notamment des triglycérides dans des globules, dont la masse peut atteindre 90% du volume cellulaire.

Ce phénomène est observable au microscope.

Or, la Société Demanderesse a le mérite d'avoir trouvé que parmi les nombreux métabolites auxquels conduit la cascade oxydative de l'acide arachidonique, le 12-HETE et le 11,12-EET identifiés plus haut, sont capables d'inhiber la différenciation des fibroblastes en adipocytes.

Ces deux métabolites constituent donc des médicaments en ce sens qu'ils restaurent, corrigent ou modifient les fonctions organiques de l'être humain.

Et l'invention a pour objet, par voie de conséquence, un médicament caractérisé par le fait qu'il comporte en tant que substance active une quantité efficace d'au moins l'un des métabolites du groupe comprenant le 12-HETE et le 11,12-EET.

Elle a plus particulièrement pour objet un médicament capable d'inhiber la différenciation de fibroblastes en adipocytes caractérisé par le fait qu'il comporte en tant que substance active une quantité efficace d'au moins l'un des acides 12-HETE et 11,12-EET.

On désigne par quantité efficace une quantité d'au moins l'un des acides 12-HETE et 11,12-EET au moins égale à celle à partir de laquelle elle est capable de restaurer, corriger ou modifier au moins une fonction organique chez l'homme ou chez l'animal et notamment d'inhiber la différenciation des fibroblastes en adipocytes.

L'invention, de par l'inhibition de la différenciation des fibroblastes en adipocytes par mise en oeuvre des métabolites12-HETE et 11,12-EET, permet de lutter contre le développement inconsidéré des couches graisseuses dont il a été question ci-dessus et, partant, contre l'installation dans l'organisme de certaines formes d'obésité.

Les métabolites 12-HETE et 11,12-EET sont disponibles dans le commerce.

Ils sont en effet commercialisés par la Société CAYMAN CHEMICAL (1180 E. Ellsworth road, Ann ARBOR, MI 48108 USA) sous les marques de fabrique 12(R)-HETE, 12(S)-HETE et (±)11 1(12)-EpEtrE.

Il est également possible de les extraire de l'algue rouge Chondrus Crispus par mise en oeuvre du procédé objet de la demande de brevet, déposée par la Société Demanderesse à la même date que la présente demande sous le titre
"Procédé de préparation d'acides gras polyinsaturés libres et leurs métabolites d'oxydation"

Ce procédé consiste essentiellement à faire agir sur l'algue une molécule stimulant ses défenses naturelles de façon à provoquer la production, entre autres, du 12-HETE et du 11,12 EET puis à les extraire, après environ 6 à 12 heures, par mise en oeuvre d'un procédé chimique d'extraction appliqué au substrat constitué par l'algue.

La propriété d'inhiber la différenciation des fibroblastes en adipocytes que présentent les métabolites 12-HETE et de 11,12EET a été démontrée par les essais in vitro décrits ci-après.

Pour ces essais in vitro, on a utilisé les lignées adipocytaires murines 3T3-F442A et BCF 115.

De plus, des cellules provenant de tissus gras humains suite à des interventions chirurgicales nécessitant leur ablation ont été utilisées. Les tissus prélevés sont collectés sur un lit de glace et traités immédiatement au laboratoire. La culture cellulaire consiste en un mélange de cellules pré-adipocytaires et adipocytaires déjà différentiées.

Les lignées préadipocytaires 3T3-F442A (lignée commerciale) et BFC 115 (provenance Claude Forest INSERM, St. Pères Paris V) sont cultivées jusqu'à la confluence dans du milieu de Eagle modifié par Dubelco (DMEM) (Gibco) en présence de 5% de sérum de veau nouveau-né et de 5% de sérum de veau foetal (Gibco).

A partir de la confluence, les cellules sont cultivées dans un milieu différenciateur (DMEM avec 10% de sérum de veau foetal et 20nM d'insuline) en présence ou non des métabolites à tester.

Le milieu est renouvelé tous les 2 ou 3 jours.

La différenciation des cellules est évaluée soit par des critères morphologiques (cellules très arrondies ayant accumulé de grosses vacuoles lipidiques) soit en mesurant la quantité moyenne de triclycérides par cellule (rapport de la quantité de triglycéride par boîte sur la quantité de protéine par boîte). Dans les conditions de culture standard, en 8 jours, 90% des préadipocytes sont différenciés en adipocytes.

Pour montrer par l'examen morphologique direct la différenciation des fibroblastes en adipocytes en présence ou non des métabolites 12-HETE et/ou 11,12-EET on a procédé comme suit.

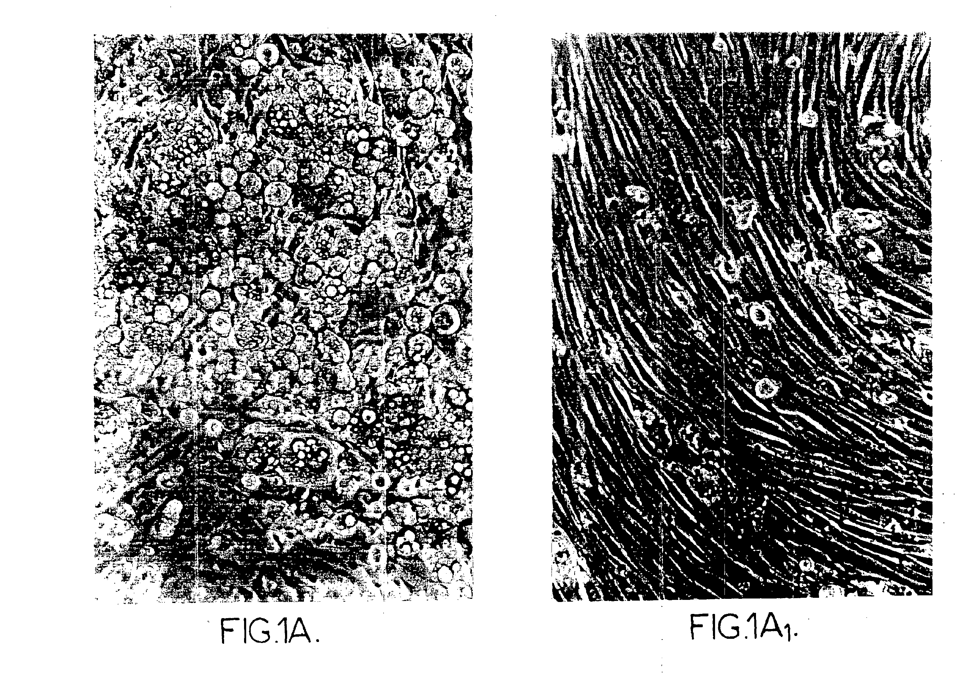

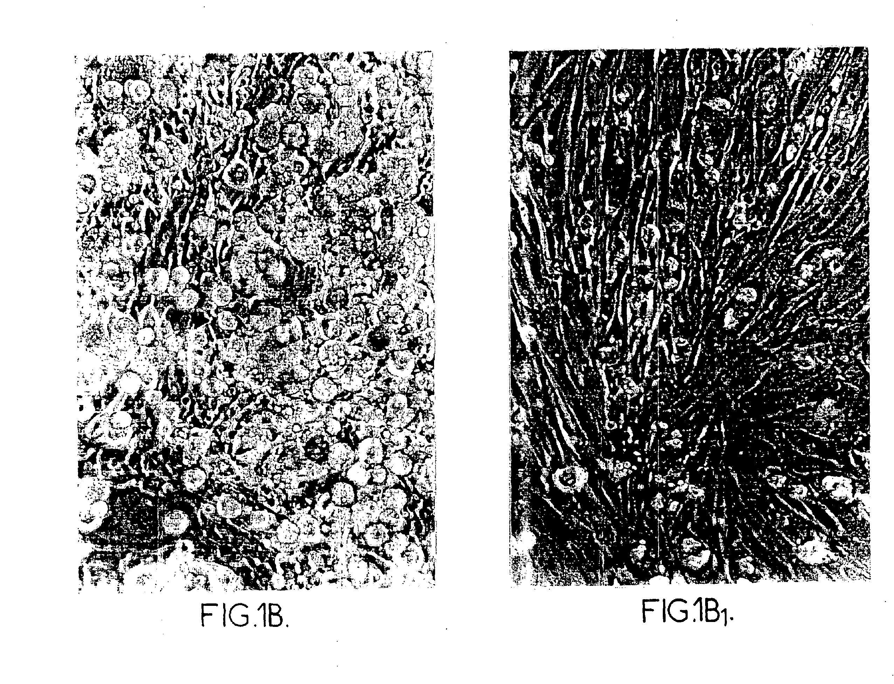

On traite les cellules 3T3-F442A à partir de la confluence avec ces métabolites. Le traitement a été renouvelé tous les 2 ou 3 jours pendant tout le processus de différenciation. Le niveau de différenciation des cellules a été évalué par observation au microscope photonique avec un grossissement de 200 fois (voir les photos montrées aux figures FIG 1A, FIG 1A₁, FIG 1B et FIG 1B₁).

La photo représentée à la figure 1A montre des cellules qui n'ont pas été mises en présence du métabolite utilisé conformément à l'invention alors que celle représentée à la figure 1A₁ montre les cellules qui ont été traitées à confluence par 10µM de 12(R)-HETE.

La photo représentée à la figure 1B montre les cellules qui ont été traitées à confluence par 100µM d'acide arachidonique et celle représentée à la figure 1B₁ les cellules traitées à confluence par 100 µM d'acide arachidonique et par 10µM de 12(R)-HETE.

L'examen des figures 1A et 1B montre une forte différenciation chez les cellules non traitées (les vacuoles lipidiques apparaissent réfringentes en blanc, figure 1A).

L'acide arachidonique augmente la quantité de cellules différenciées en adipocytes (figure 1B).

Lorsque le processus de différenciation a lieu en présence de 10µM de 12(R)-HETE, les cellules apparaissent, 7 jours plus tard, fusiformes et avec très peu de globules lipidiques (figure 1A₁) et ceci même en présence d'acide arachidonique (figure 1B₁) qui favorise la différenciation.

Cet aspect des cellules est caractéristique de cellules non différenciées.

Les cellules sont lavées au tampon phosphate PBS (Sigma), récupérées du fond de la boite de Pétri par grattage à la spatule, fragmentées aux ultra-sons (2 secondes) puis centrifugées à 10.000g pendant 20 minutes.

Le surnageant de centrifugation est prélevé pour analyse des triacylglycérides (TAG).

Pour déterminer la quantité moyenne de triclycérides par cellule à l'issue des susdites expériences, on a utilisé, comme méthode de dosage, un test colorimetrique enzymatique automatisé (Méthode Roche) qui repose sur les travaux de Wahlefeld (Wahlefeld and Bergmeyer (eds). Methods of Enzymatic Analysis. 2 second édition. New York Academic Press Inc. 1974: 1831).

Les cellules pré-adipocytaires 3T3-F442A ont été traitées a partir de la confluence par le 12(R)-HETE (10µM), le 12(S)-HETE (10 µM), le 11,12-EET (10 µM) et par la Δ15-deoxyprostaglandine J2 (10 µM, Δ 15-deoxyPGJ2) en présence ou non de 10µM de 12(R)-HETE. La différenciation des cellules a été évaluée après 7 jours en mesurant comme indiqué plus haut, la quantité de triacylglycerides accumulée par mg de protéine.

Les données ainsi obtenues, réunies dans le tableau I, illustrent les effets des stéréoisomères (S) et (R) du 12-HETE, du 11,12-EET et de la 15-deoxyPGJ2 sur la différenciation des cellules de la lignée 3T3 F442A.

**TABLEAU I**

| Traitement des cellules 3T3 F442A | TRIACYLGLYCERIDES (µg/mg protéine) |
|---|---|
| Contrôle (pas de traitement) | 0,16 |
| 12(R)-HETE (10 µM) | 0,04 |
| 12(S)-HETE (10 µM) | 0,07 |
| 11,12-EET (10 µM) | 0,08 |
| 15-deoxyPGJ2 (10 µM) | 0,52 |
| 15-deoxyPGJ2-(10 µM) plus 12(R)-HETE (10 µM) | 0,32 |

A l'examen des données réunies dans le tableau I, il apparaît
- -que les deux stéréoisomeres (S) et (R) du 12-HETE et le 11,12-EET, à une concentration de 10 µM, inhibent fortement la différenciation des cellules,
- que la capacité du 12(R)-HETE a inhiber la différenciation est supérieure à celle du 12(S)-HETE pour des concentrations identiques et
- que 10µM de 12(R)-HETE inhibent d'environ 40% la différenciation induite par la Δ15-déoxy-protaglandine J2 (PGJ2), l'inducteur naturel le plus puissant de la différenciation adipocytaire.

On a également étudié l'influence de la concentration en métabolites utilisés conformément à l'invention des solutions mises en oeuvre pour inhiber la différenciation des fibroblastes en adipocytes.

Pour ce faire, on a traité des fibroblastes ou cellules préadipocytaires 3T3-F442A à partir de la confluence par différentes concentrations en 12(R)-HETE. La différenciation des cellules a été évaluée après 7 jours en mesurant la quantité de triacylglycérides (TAG) accumulée par mg de protéine (prot).

Les concentrations mises en oeuvre étaient de 5µM, de 10µM et de 30µm.

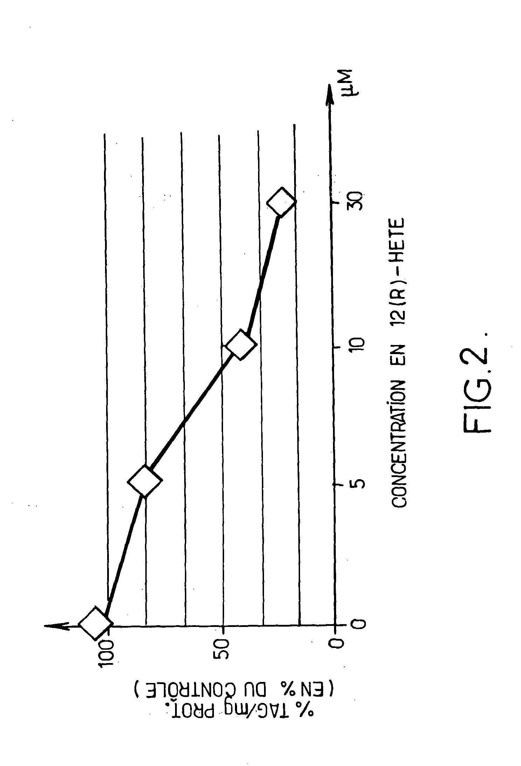

Sur le graphique de la figure 2, on a représenté la variation, en % par rapport au contrôle, de la concentration en TAG (triacylglycérides) accumulés par mg de protéine cellulaire en fonction de la concentration des solutions à base de 12(R)-HETE mises en oeuvre.

L'examen de la figure 2 permet de constater que, lorsque la concentration en 12(R)-HETE augmente de 5µM à 30µM, la concentration en TAG des adipocytes, exprimée en % par rapport au contrôle (contrôle seul : 100%) diminue d'environ 85% à environ 25%.

On a également montré que la propriété d'inhiber la différenciation des fibroblastes en adipocytes était bien attachée de façon surprenante et inattendue aux deux métabolites sélectionnés conformément à l'invention.

Pour ce faire, on a traité à la concentration de 10µM des cellules préadipocytaires de la lignée BFC-115 (lignée murine indépendante de 3T3 F442A) à partir de la confluence par les métabolites sélectionnés selon l'invention
12(R)-HETE
12(S)-HETE
11,12-EET
et par l'acide arachidonique qui favorise la différenciation.

Au bout de 7 jours on a mesuré la quantité moyenne de triacylglycérides accumulée par cellule et on a obtenu les résultats suivants, réunis dans le tableau II, ces résultats montrant les effets des stéréoisomères (S) et (R) du 12-HETE, du 11,12-EET et de l'acide arachidonique sur la différenciation des cellules de la lignée BFC-115.

Les cellules pré-adipocytaires BFC-115 ont été traitées à partir de la confluence par le 12(R)-HETE (10µM). le 12(S)-HETE (10 µM), le 11,12-EET (10 µM) et par I' acide arachidonique AA (100µM). La différenciation des cellules a été évaluée après 7 jours en mesurant la quantité de triacylglycérides accumulée par mg de protéine.

**Tableau II**

| Métabolite ou acide gras mis en oeuvre | Quantité de TAG en µg/mg de protéine |
|---|---|
| Contrôle | = 0,45 |
| 12(R)-HETE (10 µM) | = 0,17 |
| 12(S)-HETE (10 µM) | = 0,28 |
| 11,12-EET (10µM) | = 0,22 |
| AA (100µM) | = 0,55 |

A l'examen des valeurs réunies dans le tableau II, on constate que :
- les stéréoisomeres (R) et (S) du 12-HETE et le 11,12-EET à une concentration de 10 µM inhibent d'environ 40% la différenciation des pré-adipocytes de la lignée murine BFC115,
- l'acide arachidonique favorise bien la différenciation (figure 1B).

On a également déterminé que le taux résiduel de 12(R)-HETE, présent à une concentration de 10µM dans le milieu de culture des cellules 3T3-F442A différenciées, après 6h et 18h d'incubation est respectivement de 85% et 2,3%.

Les quantités résiduelles de 12(R)-HETE ont été analysées par LC-MS.

Les conclusions qu'il est permis de tirer de cet état de choses, sont que le métabolite pénètre bien dans les cellules.

Trois expériences avec des cellules de tissus adipeux humain montrent que les métabolites sélectionnés conformément à l'invention, mis en oeuvre à une concentration de 10µM, inhibent de 50% l'accumulation des triglycérides, mesurée dans une culture de cellules cultivées en milieu pro différenciateur et comparée à une culture traitée par le 12(R)-HETE et le 11.12-EET.

La toxicité éventuelle des métabolites sélectionnés conformément à l'invention a également été étudiée.

A cette fin, plusieurs marqueurs de toxicité cellulaire ont été mesurés après 8 jours de traitement, à savoir
- le nombre de cellules,
- la morphologie des cellules
- l'activité LDH (lactate déhydrogénase) dans le milieu de culture, témoin d'une perméabilisation des membranes.
- la fragmentation du noyau par coloration de "Hoesch", qui est un marqueur de l'apoptose et qui est décrit dans Leukemia, 15, 1572-81. Plenchette, S., Moutet M., Benguella M., N'Gondara J. P., Guigner F., Coffe C., Corcos L., Bettaieb A. and Solary E. (2001).

Aucun de ces marqueurs ne répond positivement pour des concentrations en 12(R)-HETE de 30µM.

En ce qui concerne la posologie et les formes pharmaceutiques d'administration pouvant être retenues pour les médicaments constitués de ou à base des métabolites sélectionnés conformément à l'invention on notera ce qui suit.

A titre d'exemples non limitatifs mais correspondant à des modes de réalisation avantageux, on indique ci-après les compositions de quelques formulations pharmaceutiques, du type désigné par l'expression Over The Counter (OTC) pharmaceutiques, dermocosmétiques, cosmétiques, et/ou nutraceutiques à savoir celles :
- d'une crème,
- d'un gel pour application locale,
- d'une gélule,
- d'un patch.

### 1- Composition d'une crème conforme à l'invention :

| | |
|---|---|
| - Eau déminéralisée | 69,50 % |
| - Glycérine | 5,00 % |
| - Acrylate | 0,20 % |
| - 12-HETE | 0,20 % |
| - PEG100 stearate | 4,00% |
| - Alcool cetearylique | 2,00 % |
| - Conservateur | 1,00 % |
| - PEG 40 stéarate | 4,00 % |
| - Acétate de vitamine E | 0,50 % |
| - C12-15 alkyl benzoate | 6,50 % |
| - Caprylic / capric triglycerides | 5,50 % |
| - Soude | 1,60% |
| | 100,00% |

### 2- Composition d'un gel pour application et massage local destiné au traitement symptomatique des surcharges adipeuses sous-cutanées localisées :

Composition pour 100 g :
- 12-HETE 5 g

Excipients : esters d'acides gras, isopropanol, polymère carboxyvinylique, diethanolamine, essences naturelles déterpénées, conservateur parahydroxybenzoate, 4-hydroxybenzoic acid methyl ester.

### 3- Composition d'une gélule à 20 mg:

Composition gélule à 20 mg:
- 12-HETE 20 mg
Excipients : lactose anhydre, stéarate de magnésium.
Enveloppe des gélules : gélatine, dioxyde de titane (E171), dioxyde de silicone, lauryl sulfate de sodium, carmin d'indigo (E 132).

### 4- Composition d'un patch dermo-cosmétique destiné à affiner la silhouette :

Composition :
- Extrait de fucus 3,00 %
- 12-HETE 2,00 %
- Vitamine C palmitate 1,00 %
- Mélange de lipides polaires 0,15 %
- Palmitate de vitamine A 0,10 %
- Extrait de Centella asiatica 3,75 %
Film support : polyuréthane, release liner : polyester.
Patch de dimension 4 x 3,5 cm et d'épaisseur 8,8 mm comme par exemple ceux développés par la Société Laboratoires LAVIPHARM Paris.

La posologie, notamment en rapport avec les susdites formes pharmaceutiques, Over The Counter (OTC) pharmaceutiques, dermopharmaceutiques, cosmétiques et/ou nutraceutique est avantageusement comme suit :
- dans le cas des crèmes, gels et sprays on prévoit 2 à 3 applications concentrées sur les surcharges adipeuses sous-cutanées localisées par jour, la durée de traitement est d'environ 5 semaines.
- dans le cas de gélules on administre 1 à 4 gélules par jour pour un adulte.
- dans le cas des patchs, le patch est appliqué tous les jours (ou tous les soirs) pendant au moins 1 mois sur les zones concernés sans stopper le traitement avant 1 mois.

## Revendications

1. Médicament **caractérisé par le fait qu'**il comporte en tant que substance active une quantité efficace d'au moins l'un des métabolites du groupe comprenant le 12-HETE et le 11,12-EET.

2. Médicament capable d'inhiber la différenciation de fibroblastes en adipocytes **caractérisé par le fait qu'**il comporte en tant que substance active une quantité efficace d'au moins l'un des acides 12-HETE et 11,12-EET.

3. Médicament selon l'une des revendications 1 et 2 **caractérisé par le fait qu'**il se présente sous la forme d'une crème, d'un gel pour application locale, d'une gélule, d'un patch.
